# EUROPÄISCHE PATENTSCHRIFT

(11) **EP 0 284 547 B1**
(45) Veröffentlichungstag und Bekanntmachung des Hinweises auf die Patenterteilung: **05.01.1994**
(21) Anmeldenummer: 88730060.6
(22) Anmeldetag: 11.03.1988
(51) Int. Cl.: C07C 405/00, A61K 31/557

(54) **6-Oxoprostaglandin-E-Derivate, Verfahren zu ihrer Herstellung und Arzneimittel**
6-Oxo prostaglandin-E derivatives, their preparation and pharmaceuticals containing them
Dérivés 6-oxo prostaglandine-E, leur préparation et médicaments les contenant

(30) Priorität: 13.03.1987 DE 3708537
(43) Veröffentlichungstag der Anmeldung: 28.09.1988
(73) Patentinhaber: SCHERING AKTIENGESELLSCHAFT, 13342 Berlin (DE)
(72) Erfinder: Klar, Ulrich, Dr., D-1000 Berlin 27 (DE); Skuballa, Werner, Dr., D-1000 Berlin 28 (DE); Vorbrüggen, Helmut, Prof., D-1000 Berlin 27 (DE); Stürzebecher, Claus-Steffen, Dr., D-1000 Berlin 46 (DE); Thierauch, Karl-Heinz, Dr., D-1000 Berlin 37 (DE); Schillinger, Ekkehard, Dr., D-1000 Berlin 28 (DE)

(56) Entgegenhaltungen:
- FR-A- 2 376 134

## Beschreibung

Die Erfindung betrifft neue 6-Oxo-prostaglandin-E-Derivate, Verfahren zu ihrer Herstellung sowie ihre Verwendung als Arzneimittel.

Aus dem sehr umfangreichen Stand der Technik der Prostaglandine insbesondere vom E-Typ und ihrer Analoga, weiß man, daß diese Stoffklasse auf Grund ihrer biologischen und pharmakologischen Eigenschaften zur Therapie und Prophylaxe von Thrombosen, Infarkten und anderer Herz-Kreislauf-Erkrankungen geeignet sind. Strukturveränderungen haben deshalb zum Ziel, die Wirkungsdauer zu verlängern, die Selektivität der Wirksamkeit zu steigern und gleichzeitig die Wirkungsdosis zu reduzieren.

Es wurde nun Überraschenderweise gefunden, daß durch die Einfuhrung einer Dreifachbindung in die 18, 19- oder 19, 20- und/oder 13, 14-Position sowie die Einführung einer Methylgruppe in die 16- und/oder 20-Position der unteren Kette der 6-Oxoprostaglandin-E₁-Analoga die Wirksamkeit verbessert, die Selektivität erhöht und die Wirkungsdauer verlängert werden kann.

Die erfindungsgemäßen Verbindungen wirken thrombozytenaggregationshemmend, blutdrucksenkend, vaso- und bronchodilatatorisch. Sie sind außerdem zur Inhibierung der Magensäuresekretion sowie zur Cytoprotektion an Magen, Herzen, Leber, Pankreas und Niere geeignet.

Die erfindung betrifft 6-Oxo-prostaglandin-E₁-Derivate der Formel I, 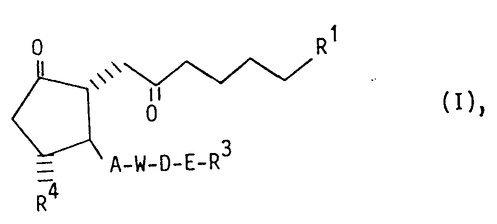
worin
- R¹: den Rest COOR² mit R² in der Bedeutung eines Wasserstoffatomes, einer C₁₋₁₀-Alkyl-, einer gegebenenfalls durch Alkylgruppen mit 1-4 C-Atomen substituierten C₅₋C₆-Cycloalkyl- oder einer C₆₋C₁₀-Aryl-Gruppe oder eines heterocyclischen Restes, oder den Rest CONHSO₂ R⁵ mit R⁵ als C₁₋₁₀-Alkyl- gegebenfalls durch Alkylgruppen mit 1-4 C-Atomen substituierte C₅₋₆-Cycloalkyl- oder C₆₋₁₀-Aryl-Gruppe,
- A: eine E-konfigurierte CH=CH- oder eine -C≡C-Gruppe,
- W: eine freie oder funktionell abgewandelte 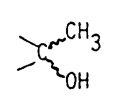 Hydroxymethylengruppe oder eine freie oder funktionell abgewandelte -Gruppe, wobei die OH-Gruppe jeweils α- oder β-ständig sein kann,
- D: eine geradkettige oder verzweigtkettige Alkylengruppe mit 1-5 C-Atomen und
- E: eine -C≡C-Gruppe in 18, 19- oder 19, 20- Position,
- R³: eine gesättigte oder ungesättigte C₁-C₁₀ -Alkyl, gegebenenfalls durch Alkylgruppen mit 1-4 C-Atomen substituierte C₃-C₁₀ -Cycloalkyl- oder eine gegebenenfalls substi tuierte C₆-C₁₀ -Arylgruppe oder eine heterocyclische Gruppe,
- R⁴: eine freie oder funktionell abgewandelte Hydroxygruppe, wobei im 16- und/oder 20 Position Methyl steht, und
falls R² die Bedeutung eines Wasserstoffatoms hat, deren Salze mit physiologisch verträglichen Basen bedeuten sowie die α-, β- oder γ-Cyclodextrin clathrate der Verbindungen der Formel I.

Als Alkylgruppen R² und R⁵ sind gerad- oder verzweigtkettige Alkylgruppen mit 1-10 C-Atomen gemeint wie beispielsw. Methyl, Ethyl, Propyl, Isopropyl, Butyl, Isobutyl, tert.-Butyl, Pentyl,Isopentyl,Neopentyl,Heptyl,Hexyl,Decyl.

Als bevorzugte Alkylgruppen R² sind solche mit 1-4 C-Atomen wie z.B. Methyl, Ethyl, Propyl, Isobutyl und Butyl zu nennen.

Als Arylgruppen R² und R⁵ kommen unsubstituierte Arylgruppen in Betracht wie beispielsw. Phenyl-, α- oder β-Naphthyl.

Die Cycloalkylgruppen R² und R⁵ können im Ring 5 und 6 Kohlenstoffatome enthalten. Die Ringe können durch Alkylgruppen mit 1-4 Kohlenstoffatomen substituiert sein. Als Beispiele seien Cyclopentyl-, Cyclohexyl-, Methylcyclohexyl- und Adamantyl genannt.

Als heterocyclische Gruppen R² kommen 5- und 6-gliedrige Heterocyclen in Frage, die bevorzugt ein Heteroatom, vorzugsweise Stickstoff, Sauerstoff oder Schwefel enthalten. Als Beispiele seien 2-Furyl, 3-Furyl, 2-Thienyl, 3-Thienyl, 2-Pyridyl, 3-Pyridyl und 4-Pyridyl genannt.

Die Hydroxygruppen R⁴ und in W können funktionell abgewandelt sein, beispielsweise durch Veretherung oder Veresterung, wobei die freien oder abgewandelten Hydroxygruppen in W α- oder β-standig sein können und freie Hydroxygruppen bevorzugt sind.

Als Ether und Acylreste kommen die dem Fachmann bekannten Reste in Betracht. Bevorzugt sind leicht abspaltbare Etherreste, wie beispielsweise der Tetrahydropyranyl-, Tetrahydrofuranyl-, Methoxymethyl-, Methoxy ethyl-, tert.-Butyldimethylsilyl-, tert.-Butyl-diphenylsilyl-, Thexyl-dimethylsilyl-und α-Tribenzyl-silylrest. Als Acylreste seien beispielsweise Acetyl, Propionyl, Butyryl und Benzoyl genannt.

Als Alkylgruppen R³ kommen gerad- und verzweigtkettige, gesittigte und ungesättigte Alkylreste, vorzugsweise gesättigte, mit 1-10, insbesondere 1-7 C-Atomen in Frage, Als Beispiele seien Methyl, Ethyl, Propyl, Butyl-, Isobutyl, tert.-Butyl, Pentyl, Hexyl, Heptyl, Octyl, Butenyl, Isobutenyl, Propenyl, Pentenyl und Hexenyl genannt.

Die Cycloalkylgruppe R³ kann im Ring 3-10, vorzugsweise 3-6 Kohlenstoffatome enthalten. Die Ringe können durch Alkylgruppen mit 1-4 Kohlenstoffatomen substituiert sein. Als Beispiele seien Cyclopropyl, Cyclobutyl, Cyclopentyl, Cyclohexyl, Methylcyclohexyl und Adamantyl genannt.

Als substituierte bzw. unsubstituierte Arylgruppen R³ kommen beispielsweise Phenyl, 1-Naphthyl und 2-Naphthyl, die jeweils durch 1-3 Halogenatome, eine Phenylgruppe, 1-3 Alkylgruppen mit jeweils 1-4 Kohlenstoffatomen, eine Chlormethyl-, Fluormethyl-, Trifluormethyl-, Carboxyl-, C₁-C₄ -Alkoxy oder Hydroxygruppe substituiert sein können, in Betracht. Bevorzugt ist die Substitution in 3- und 4-Position am Phenylring z. B. durch Fluor, Chlor, C₁-C₄ -Alkoxy oder Trifluormethyl oder in 4-Position durch Hydroxy.

Als heterocyclische Gruppen R³ kommen 5- und 6-gliedrige Heterocyclen, die wenigstens 1 Heteroatom, vorzugsweise Stickstoff, Sauerstoff oder Schwefel enthalten, in Frage. Als Beispiele seien 2-Furyl, 3-Furyl, 2-Thienyl, 3-Thienyl, 2-Pyridyl, 3-Pyridyl und 4-Pyridyl genannt.

Als Alkylengruppe D kommen geradkettige oder verzweigtkettige, gesättigte Alkylenreste, vorzugsweise gesättigte mit 1-5 C-Atomen, in Frage,. Als Beispiele seien Methylen, Ethylen, 1.2-Propylen, Ethylethylen, Trimethylen, Tetramethylen, Pentamethylen, 1-Methyltetramethylen und 1-Methyl-trimethylen genannt.

Zur Salzbildung mit den freien Säuren (R² = H) sind anorganische und organische Basen geeignet, wie sie dem Fachmann zur Bildung physiologisch verträglicher Salze bekannt sind. Als Beispiele seien Alkalihydroxide wie Natrium- und Kaliumhydroxid, Erdalkalihydroxide wie Calciumhydroxid, Ammoniak, Amine, wie Ethanolamin, Diethanolamin, Triethanolamin, N-Methylglucamin, Morpholin-, Tris-(hydroxymethyl)- methylamin etc. genannt.

Die Erfindung betrifft ferner ein Verfahren zur Herstellung der Verbindungen der Formel I, das dadurch gekennzeichnet ist, daß man eine Verbindung der Formel II 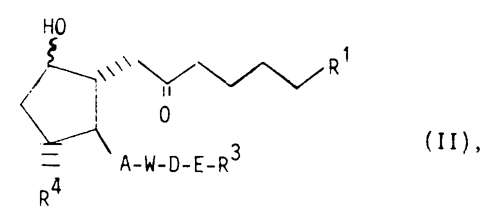
worin R¹, R³, R⁴, A, W, D und E die obenangegebenen Bedeutungen aufweisen und freie OH-Gruppen in R⁴ und W geschützt sind, mit dem Reagenz Chromschwefelsäure (Jones-Reagenz, J. Chem. Soc. 1953, 2555), Pyridiniumdichromat (Tetrahedron Lett. 1979,399), Pyridiniumchlorochromat (Tetrahedron Lett. 1975, 2647), Collins-Reagenz oder der Komplexe aus CrO₃ mit anderen Aminbasen wie z.B. Pyrazol und Benzotriazol zu einer Verbindung der Formel I oxidiert und gegebenenfalls geschützte Hydroxygruppen freisetzt und/oder freie Hydroxygruppen verestert, verethert und/oder eine veresterte Carboxygruppe verseift oder eine Carboxygruppe mit einer physiologisch verträglichen Base in ein Salz oder mit α-, β oder γ-Cyclodextrin in ein Clathrat überführt.

Die Umsetzung der Verbindungen der allgemeinen Formel II zu den Verbindungen der allgemeinen Formel I wird mit Jones-Reagenz bei -40°C bis 0°C, vorzugsweise bei -30°C bis -10°C, unter Verwendung der anderen Oxidationsverfahren vorzugsweise bei -10°C bis +25°C durchgeführt. Als Lösungsmittel eignen sich Methylenchlorid, Chloroform, Ethylenchlorid, Aceton, Pyridin, u.a., vorzugsweise jedoch Methylenchlorid und Aceton.

Die Freisetzung der funktionell abgewandelten Hydroxygruppen R⁴ und in W zu den Verbindungen der allgemeinen Formel I erfolgt nach den dem Fachmann bekannten Methoden. Beispielsweise wird die Abspaltung von Etherschutzgruppen in einer wässrigen Lösung einer organischen Säure, wie z.B. Essigsäure, Propionsäure, Zitronensäure u.a. oder in einer wässrigen Lösung einer anorganischen Säure, wie z.B. Salzsäure, oder im Falle von Tetrahydropyranylethern unter Verwendung von Pyricinium-p-Toluolsulfonat, vorzugsweise in Alkoholen als Lösungsmittel oder unter Verwendung von wasserfreiem Magnesiumbromid, vorzugsweise in Diethylether als Lösungsmittel, durchgeführt.

Zur Verbesserung der Löslichkeit wird bei Verwendung wässrig-saurer Reaktionsbedingungen zweckmäßigerweise ein mit Wasser mischbares inertes Lösungsmittel zugesetzt. Als geeignet erweisen sich z.B. Alkohole wie Methanol und Ethanol, Ether wie Dimethoxyethan, Dioxan und Tetrahydrofuran, wobei Tetrahydrofuran bevorzugt angewendet wird.

Die Abspaltung der Silyletherschutzgruppen erfolgt beispielsweise mit Tetrabutylammoniumfluorid. Als Lösungsmittel sind beispielsweise Tetrahydrofuran, Diethylether, Dioxan, Methylenchlorid etc. geeignet.

Die Abspaltung wird vorzugsweise bei Temperaturen zwischen 20°C und 80°C durchgeführt.

Die Verseifung der Acylgruppen und Prostaglandinester wird nach den dem Fachmann bekannten Methoden durchgeführt, wie beispielsweise mit basischen Katalysatoren wie z.B. mit Alkali- oder Erdalkali-carbonaten oder -hydroxiden in einem Alkohol oder in der wässrigen Lösung eines Alkohols. Als Alkohole kommen aliphatische Alkohole wie z.B. Methanol, Ethanol, Butanol etc. in Betracht, vorzugsweise jedoch Methanol. Als Alkalicarbonate und -hydroxide seien Kalium- und Natriumsalze genannt. Bevorzugt sind die Kaliumsalze. Als Erdalkalicarbonate und -hydroxide eignen sich beispielsweise Calciumcarbonat, Calciumhydroxid und Bariumcarbonat. Die Umsetzung erfolgt allgemein bei -10°C bis +70°C, vorzugsweise jedoch bei +25°C.

Die Einführung der Estergruppe CO₂R² für R¹, bei welcher R² eine Alkylgruppe mit 1-10 C-Atomen darstellt, erfolgt nach den dem Fachmann bekannten Methoden. Die 1-Carboxyverbindungen (R² = H) werden beispielsweise mit Diazokohlenwasserstoffen in an sich bekannter Weise umgesetzt. Die Veresterung mit Diazokohlenwasserstoffen erfolgt z.B. dadurch, daß eine Lösung des Diazokohlenwasserstoffes in einem inerten Lösungsmittel, vorzugsweise in Diethylether, mit der 1-Carboxyverbindung, gelöst in dem gleichen oder in einem anderen ebenfalls inerten Lösungmittel, wie z.B. Methylenchlorid, vermischt wird. Nach beendeter Umsetzung in 1 bis 60 Minuten wird das Lösungsmittel entfernt und der Ester in üblicher Weise gereinigt. Diazoalkane sind entweder bekannt oder können nach bekannten Methoden hergestellt werden [ Org. Reactions Bd. 8, Seiten 389-394 (1954)].

Die Einführung der Amidgruppe CONHSO₂R⁵ für R₁ erfolgt nach den dem Fachmann bekannten Methoden. Die Carbonsäuren der allgemeinen Formel I (R₂=H) werden zunächst in Gegenwart eines tertiären Amins, wie beispielsweise Triäthylamin, mit Chlorameisensäureisobutylester in das gemischte Anhydrid überführt. Die Umsetzung des gemischten Anhydrids mit dem Alkalisalz des entsprechenden Amids oder mit Ammoniak (R₃=H) erfolgt in einem inerten Lösungsmittel oder Lösungsmittelgemisch, wie beispielsweise Tetrahydrofuran, Dimethoxyäthan, Dimethylformamid, Hexamethylphosphorsäureretriamid, bei Temperaturen zwischen -30° C und + 60° C, vorzugsweise bei 0° C bis 30°C.

Die Einfuhrung der Estergruppe CO₂R² für R¹, bei welcher R² eine substituierte oder unsubstituierte Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base wie z.B. Pyridin, Dimethylaminopyridin, Triethylamin, in einem inerten Lösungsmittel wie z.B. Methylenchlorid, Ethylenchlorid, Chloroform, Essigsäureethylester, Tetrahydrofuran, vorzugsweise jedoch Chloroform umgesetzt. Die Reaktion wird bei Temperaturen zwischen -30°C und +50°C vorzugsweise bei +10°C, durchgeführt.

Die Prostaglandinderivate der Formel I mit R¹ in der Bedeutung eines Wasserstoffatoms können mit geeigneten Mengen der entsprechenden anorganischen Basen unter Neutralisierung in Salze überführt werden. Beispielsweise erhält man beim Lösen der entsprechenden Prostaglandinsäuren in Wasser, welches stöchiometrische Mengen der Base enhält, nach Abdampfen des Wassers oder nach Zugabe eines mit Wasser mischbaren Lösungsmittels, z.B. Alkohol oder Aceton, das feste anorganische Salz.

Die Herstellung der Aminsalze erfolgt in üblicher Weise. Dazu löst man die Prostaglandinsaüre in einem geeigneten Lösungsmittel, wie z.B. Ethanol, Aceton, Diethylether oder Benzol und setzt 1 bis 5 Äquivalente des jeweiligen Amins dieser Lösung zu. Dabei fällt das Salz gewöhnlich in fester Form an oder wird nach Verdampfen des Lösungsmittels in üblicher Weise isoliert.

Die funktionelle Abwandlung der freien Hydroxygruppen erfolgt nach den dem Fachmann bekannten Methoden. Zur Einführung der Etherschutzgruppen wird beispielsweise mit Dihydropyran oder Methylvinylether in Methylenchlorid oder Chloroform unter Verwendung katalytischer Mengen eines sauren Kondensationsmittels wie z.B. p-Toluolsulfonsaüre, umgesetzt. Der jeweilige Enolether wird im Überschuß, vorzugsweise in der 1.5- bis 10-fachen Menge des theoretischen Bedarfs, zugesetzt. Die Umsetzung erfolgt normalerweise bei -10°C bis +30°C und ist nach 2-30 Minuten beendet.

Die Einführung der Acylschutzgruppen erfolgt, indem man eine Verbindung der Formel I in an sich bekannter Weise mit einem Carbonsiurederivat, wie z.B. Säurechlorid, Säureanhydrid etc., umsetzt.

Die Verbindungen der allgemeinen Formel I wirken blutdrucksenkend und bronchodilatorisch. Sie eignen sich zur Hemmung der Thrombozytenaggregation, wirken cytoprotektiv an Magen, an der Leber, der Niere und am Pankreas und sind deshalb auch bei Organtransplantationen anwendbar. Demzufolge stellen die neuen 6-Oxo-prostaglandin-E-derivate wertvolle pharmazeutische Wirkstoffe dar. Darüberhinaus weisen sie bei ähnlichem Wirkungsspektrum, verglichen mit entsprechenden Prostaglandinen eine höhere Spezifität und längere Wirkdauer auf.

Die neuen Prostaglandin-Analoga besitzen die für Prostaglandin E typischen Eigenschaften, wie z.B. Senkung des peripheren arteriellen und des koronaren Gefäßwiderstandes, Inhibierung der Thrombozytenaggregation und Auflösung von Plättchenthromben, myocardiale Cytoprotektion und somit Senkung des systemischen Blutdruckes ohne zugleich Schlagvolumen und koronare Durchblutung zu senken; weitere Indikationen können prinzipiell sein: Schlaganfall, Prophylaxe und Therapie koronarer Nerzarkrankungen, koronarer Thrombose, des Herzinfarktes, peripherer Arterienerkrankungen, Arteriosklerose und Thrombose, Asthma, Prophylaxe und Therapie ischaemischer Attacken des ZNS-Systems, Therapie des Schocks, Inhibierung der Bronchokonstriktion, Inhibierung der Magensäuresekretion, Zytoprotektion der Magen- und Darmschleimhaut, Zytoprotektion in der Leber, Niere und im Pankreas, Senkung des pulmonalen vaskulären Widerstandes und des pulmonalen Blutdruckes, Förderung der Nierendurchblutung, Anwendung anstelle von Heparin oder als Adjuvans bei der Dialyse oder der Hämofiltration, Konservierung von Blut- und Thrombozyten-Konserven, Transplantaten, Inhibierung von Geburtgswehen, Erhöhung der zerebralen Durchblutung, Glaukom-Behandlung, Einbau in künstliche Gefäße, chirurgisches Nahtmaterial, Venenkatheter, etc.

Die 6-Oxo-Prostaglandin-E-Derivate dieser Erfindung können auch in Kombination, z.B. mit β-Blockern, Diuretika, Phosphodiesterasehemmern, Ca-Antagonisten,t-PA, nichtsteroidalen Entzündungshemmern, Leukotriensynthetasehemmern, Leukotrienantagonisten, Thromboxansynthetasehemmern oder Thromboxanantagonisten verwendet werden.

Die Dosis der Verbindungen ist 1-1000 µg/kg/Tag, wenn sie am menschlichen Patienten verabreicht wird. Die Einheitsdosis für den pharmazeutisch akzeptablen Träer beträgt 10 µg bis 100 µg.

In der nachfolgenden Tabelle werden die pharmakologischen Vergleichsversuche, die mit den Verbindungen aus Beispiel 1 und 4 im Vergleich zu 6-Oxo-PGE₁ durchgeführt wurden, zusammengefaßt.

| Verbindung | Thrombozytenaggregationshemmung IC₅₀ | Thrombozyten Shape-change IC₅₀ | Blutdrucksenkung SH-Ratte | Zytoprotektion Magen(Ratte,Schädigung mit Ethanol) |
|---|---|---|---|---|
| 6-OxoPGE₁ | 7x10⁻⁹m | | Dosis: 10µg/kg | |
| | | | Pₛ min: 99 → 73 | |
| | | | P_{D} min: 99 → 44 | |
| | | | HF :100 → 139 | |
| | | | (Herzfrequenz) | |
| Beispiel 1 | 1.7x10⁻⁹m | 1x10⁻⁸m | Dosis: 3µg/kg | 52% Hemmung (10µg/kg) |
| | | | Pₛmin: 92 → 64 | |
| | | | P_{D}min: 93 → 37 | |
| | | | HF : 107 → 149 | |
| Beispiel 4 | 9x10⁻¹¹m | 1x10⁻⁹m | | 86% Hemmung (10µ/kg) |

Für die parenterale Applikation werden sterile, injizierbare wäßrige oder ölige Losungen benutzt. Fur die orale Applikation sind beispielsweise Tabletten, Dragees oder Kapseln geeignet. Die Erfindung betrifft somit auch Arzneimittel auf Basis der Verbindungen der Formel I und üblicher Hilfs- und Trägerstoffe einschließlich Cyclodextrinclathrate.

Die erfindungsgemäßen Wirkstoffe sollen in Verbindung mit den in der Galenik bekannten und üblichen Hilfsstoffen zum Beispiel zur Herstellung von Blutdrucksenkern, Thrombozytenaggregationshemmern oder Cytoprotektiva dienen.

### Beispiel 1

### (13 E) - (11 R, 15 S, 16 RS) - 6,9 - Di - oxo - 11,15 - dihydroxy - 16 - methyl - 18,18,19,19 - tetradehyro - 13 - prostensäure

160 mg (13 E) - (11 R, 15 S, 16 RS) - 6,9 - Di - oxo - 11,15 - bis - (tetrahydropyran - 2 - yloxy) - 16-methyl) - 18,18,19,19 - tetradehydro13 - prostensäure versetzte man mit 88 ml eines Gemisches aus Essigsäure : Wasser : Tetrahydofuran (65 : 35 : 10) und ließ 15 h bei Raumtemperatur regieren. Man engte im Vakuum ein und entfernte durch Zusatz von Toluol mittels mehrfach wiederholter azeotroper Vakuumdestillationen Restanteile an Essigsäure und Wasser. Das erhaltene Rohöl reinigte man durch Chromatographie an mit Kieselgel beschichteten Glasplatten. Als Laufmittel diente ein Gemisch aus Dichlormethan und Methanol. Isoliert wurden 108 mg (98 %) der Tritelverbindung als farbloses Öl.
- IR (Film):: 3380, 3600 - 2400, 2960, 2920, 2870, 1740, 1725, 1710, 1565, 1405, 1285, 1158, 1080, 1020, 973 cm⁻¹.

### Beispiel 2

### (13 E) - (11 R, 15 S, 16 RS) - 6,9 - Di - oxo - 11,15 - bis - (tetrahydropyran - 2 - yloxy) - 16 - methyl - 18,18,19,19 - tetradehydro - 13 - prostensäure

Die Lösung von 396 mg (13 E) - (9 S, 11 R, 15 S, 16 RS) - 6 - Oxo - 9 - hydroxy - 11,15 - bis - (tetrahydropyran - 2 - yloxy) - 16 - methyl - 18, 18,19,19 - tetradehydro - 13 - prostensäure in 8 ml abs. Dimethylketon kühlte man unter Feuchtigkeitsausschluß auf - 30° C , versetzte mit 270 µl Jones-Lösung und rührte 1,5 h bei - 30°C bis - 20°C. Nach Zugabe von 2 ml Isopropanol goß man auf 50 ml Eiswasser, extrahierte mehrmals mit insgesamt 100 ml Diethylether, wusch mit ges. Natriumchloridlösung neutral, trocknete über Magnesiumsulfat, filtrierte und engte im Vakuum ein. Das farblose Rohöl chromatographierte man an mit Kieselgel beschichteten Platten mit HexanEthylacetat. Man isolierte neben Ausgangsmaterial 160 mg (41 %) der Titelverbindung als farbloses Öl.
- IR (Film):: 3600 - 2500, 2940, 2870, 1742, 1726, 1710, 1452, 1440, 1380, 1380, 1352, 1200, 1125 (breit), 1078, 1035, 1022, 972, 912, 870, 816 cm⁻¹.

Das Ausgangsmaterial wurde wie in Beispielen 2 a bis 2 h beschrieben dargestellt.

### Beispiel 2 a

### (13 E) - (9 S, 11 R, 15 S, 16 RS) - 6 - Oxo - 9 - hydroxy - 11,15 - bis - (tetrahydropyran - 2 - yloxy) - 16 - methyl - 18,18,19,19 - tetradehydro - 13 - prostensäure

690 mg (5 RS, 6 RS, 16 RS) - 5 - Iod - 16 - methyl - 18,18,19,19 - tetradehydro - prostaglandin I₁ - 11,15 - bis - (tetrahydropyranylether) - methylester löste man in 30 ml abs. Benzol, versetzte mit 2,3 ml Diazabicyloundecan und erhitzte unter Feuchtigkeitsauzschluß 2 h auf 50° C. Die erkaltete Lösung verdünnte man mit 60 ml Ethylacetat, wusch zweimal mit einer ges. Natriumhydrogencarbonatlösung und trocknete die organische Phase über einem Gemisch aus Magnesiumsulfat und Kaliumcarbonat. Nach Filtration und Eindamfen im Vakuum wurde das erhaltene Rohöl in 25 ml Methanol aufgenommen, mit der Losung von 600 mg Kaliumhydroxid in 5 ml Wasser versetzt und 16 h gerührt. Man engte auf 5 ml im Vakuum ein, verdünnte mit 70 ml Wasser und extrahierte mit 50 ml Ether. Die abgetrennte organische Phase wusch man mit einer 2n Natronlauge nach, säuerte die vereinigten basischen Extrakte mit ges. Citronensäure auf pH 4.5 an und extrahierte mehrmals mit insgesamt 100 ml Trichlormethan. Die organischen Extrakte wurden mit ges. Natriumchloridlösung neutral gewaschen, über Magnesiumsulfat getrocknet, filtriert und im Vakuum eingeengt. Man erhielt 609 mg eines gelben Öles das mit Ethylacetat-Isopropanol an Kieselgel chromatographiert wurde. Man isolierte als Hauptkomponente 396 mg (70 %) der Titelverbindung als farbloses Öl.
- IR (Film):: 3420, 3600- 2500, 2940, 2870, 1730, 1710, 1450, 1440, 1382, 1350, 1200, 1125 (breit), 1075, 1020, 973, 908, 868, 813 cm⁻¹.

### Beispiel 2 b

### (5 RS, 6 RS, 16 RS) - 5 - Iod - 16 - methyl - 18,18,19,19 - tetradehydroprostaglandin I₁ - 11,15 - bis - (tetrahydropyranylether) - methylester.

Die Lösung von 1,72 g (5 E/Z, 13 E) - (9S, 11 R, 15 S, 16 RS) - 9 - Hydroxy - 11,15 - bis - (tetrahydropyran - 2 - yloxy) - 16 - methyl - 18,18,19,19 - tetradehydro - prostadiensäure - methylester in 50 ml Diethylether versetzte man mit der Lösung von 4,10 g Natriumhyrogencarbonat in 70 ml Wasser, kühlte auf 0 - 5° C und ließ innerhalb 90 min die Losung von 1,77 g Iod in 65 ml Diethylether zutropfen. Man ließ noch 3 h bei 0 - 5° C reagieren, reduzierte überschüssiges Iod durch Zugabe entsprechender Mengen einer ca. 20 %igen Natriumthiosulfatlösung, trennte die organische Phase ab und wusch mit ges. Natriumchloridlösung. Nach dem Trocknen über Magnesiumsulfat, Filtration und Eindampfen im Vakuum isolierte man 2,11 g (100 %) der Titelverbindung als gelbes Öl.
- IR (Film):: 2970, 2870, 1738, 1450, 1438, 1200, 1120 (breit), 1075, 1034, 1020, 974, 907, 868, 815 cm⁻¹.

### Beispiel 2 c

### (5 Z, 13 E) - (9 S, 11 R, 15 S, 16 RS) - 9 - Hydroxy - 11,15 - bis - (tetrahydropyran - 2 - yloxy) - 16 - methyl - 18,18,19,19 - tetradehydro-prostadiensäure - methylester

Die Lösung von 16,54 g carboxybutyltriphenylphosphoniumbromid in einem Gemisch aus 35 ml abs. Dimethylsulfoxid und 15 ml abs. Tetrahydrofuran kühlte man auf 3° C und versetzte unter Feuchtigkeitsausschluß portionsweise mit insgesamt 8,0 Kalium - tert.-butanolat. Anschließend ließ man innerhalb 1 Stunde die Lösung von 1,81 g (1 S, 3 RS, 5 R, 6 S, 7 R) - 7 - (Tetrahydropyran - 2 - yloxy) - 6[(13E, 3 S, 4 RS) - 4 - methyl - 3 - (tetrahydropyran - 2 - yloxy) - 1 - octen - 6 inyl)] - bicyclo [3.3.0] octan - 3 - ol in 200 ml abs. Tetrahydrofuran zutropfen, auf Raumtemperatur erwärmen und noch weitere 30 min - reagieren. Unter kräftigem Rührem goß man in 200 ml eiskaltes Wasser, stellte mit einer 1 n HCL auf pH 3 ein und extrahierte mehrmals mit insgesamt 200 ml Diethylether. Die organische Phase wusch man mit ges. Natriumchloridlösung neutral, trocknete über Magnesiumsulfat und filtrierte ab. Das Filtrat versetzte man mit einer etherischen Lösung von Diazomethan, filtrierte erneut und dampfte im Vakuum ein. Nach Chromatographie des Rückstandes an Kieselgel mit Hexan/Ethylacetat isolierte man 1,72 g (78 %) der Titelverbindung neben Spuren des 5 E - Isomeren als farbloses Öl.
- IR (Film):: 3450, 2940, 2870, 1738, 1450, 1437, 1200, 1130, 1077, 1021, 974, 907, 868, 813 cm⁻¹.

### Beispiel 2 d

### (1 S, 3 RS, 5 R, 6 S, 7 R) - 7 - (Tetrahydropyran - 2 - yloxy) - 6-[(13E, 3 S,4 RS) - 4 - methyl - 3 - (tetrahydropyan - 2 - yloxy - 1 octen - 6 - inyl)] - 2 - oxa - bicyclo [3.3.0] octan - 3 - ol

Bei - 70° C versetzte man die Lösung von 1,98 g Lacton in 60 ml abs. Toluol mit 15 ml einer 1 M Lösung von Diisobutylaluminiumhydrid in Toluol und rührte 50 min. bei - 65° C unter einer Atmosphäre aus trockenem Argon. Nach Zusatz von 1.3 ml Isopropanol und anschließend 10 ml Wasser, ließ man auf Raumtemperatur erwärmen, saugte von dem feinkörnigen Niederschlag ab und engte im Wasserstrahlvakuum ein. Man isolierte 1,81 g (91 %) eines farblosen Öles, das ohne Reinigung weiter umgesetzt wurde.

### Beispiel 2 e

### (1 S, 5 R, 6 S, 7 R) - 6 - [(E) - (3 S, 4 RS)- 4 - Methyl - 3 - (tetrahydropyran - 2 - yloxy) - oct - 1 - en - 6 - inyl] - 7 - (tetrahydropyran - 2 - yloxy) - 2 - oxabicyclo [3.3.0] octan - 3 - on

Die Lösung aus 2.16 g (1 S, 5 R, 6 S, 7 R) - 6-[(E) - (3 S, 4 RS) - 3 - Hydroxy - 4 - methyl - oct - 1 - en - 6 - inyl] - 7 - hydroxy - 2 - oxa - bicyclo [3.3.0] octan - 3 - on in 50 ml wasserfreiem Methylenchlorid versetzte man mit 1,7 ml Dihydropyran, einer Microspatelspitze p - Toluolsulfonsäure und rührte 30 Minuten bei 25° C unter einer Atmosphäre aus trockenem Argon. Man versetzte mit 20 ml einer 10 %igen wässrigen Bicarbonatlösung, trennte die organische Phase ab, wusch mit Wasser nach und trocknete über Magnesiumsulfat. Das Rohprodukt chromatographierte man mit einem Gradientensystem aus Hexan/Essigsäureethylester an Kieselgel und isolierte 2,96 g (85 %) der Titelverbindung als farbloses Öl.
- IR (CHCl₃):: 2945, 2870, 1767, 1452, 1440, 1352, 1261, 1182, 1128, 1074, 1020, 973, 910, 870, 869, 811 cm⁻¹.

### Beispiel 2 f

### (1 S, 5 R, 6 S, 7 R) - 6- [(E)- (3 S, 4 RS) - 3 - Hydroxy - 4 - methyl - oct - 1 - en - 6 - inyl ] - 7- hydroxy - 2 - oxabicyclo [ 3.3.0] octan - 3 - on

Die Lösung aus 4,78 g (1 S, 5 R, 6 S, 7 R) - 6 - [(E) - (3 S, 4 RS) - 3 - Hydroxy - 4 - methyl - oct - 1 - en - 6 - inyl ] - 7 - benzoyloxy - 2 - oxabicyclo - [3.3.0] octan - 3 - on in 50 ml wasserfreiem Methanol versetzte man mit 2.1 g fein gepulvertem Kaliumcarbonat und rührte 5 h bei 25° C unter einer Atmosphäre aus trockenem Argon. Mit einer 10%igen wäsrigen Citronensäurelösung stellte man pH 7 ein, engte im Vakuum auf 60 ml Restvolumen ein, versetzte mit 100 ml Wasser und extrahierte mehrfach mit 300 ml Dichlormethan. Die vereinigten organischen Phasen wusch man mit Wasser, trocknete über Magnesiumsulfat und dampfte nach Filtration im Vakuum ein. Das gelbe Rohöl chromatographierte man unter Druck an einer Kieselgelsäule mit einem Gradientengemisch aus Hexan und Aceton. Man isolierte 2,96 g (85 %) der Titelverbindung als farbloses Öl.
- IR (Film):: 3350, 2960, 2870, 1760, 1640, 1435, 1420, 1350, 1180, 1075, 1020, 970, 908 cm⁻¹.

### Beispiel 2 g

### (1 S, 5 R, 6 S, 7 R) - 6 - [(E) - (3 S, 4 RS) - 3 - Hydroxy - 4 - methyl - oct - 1 - en - 6 - inyl ] -7 - benzoyloxy -2 - oxabicyclo - [3.3.0] octan - 3 - on

Die Lösung von 12.5 g (1 S, 5 R, 6 S, 7 R) - 6 - [(E) - (4 RS) - 3 - Oxo - 4 - methyl - oct - 1 - en - 6 - inyl] - 7 - benzoyloxy - 2 - oxabicyclo [3.3.0] octan - 3 - on in einem Gemisch aus 300 ml Methanol und 80 ml Tetrahydrofuran wird unter Feuchtigkeitsausschluß bei -40° C mit 1.84 g CeCl₃. 7 H₂O und anschließend portionsweise mit insgesamt 1.85 g Natriumborhydrid versetzt. Nach 1 h bei -40° C wurde mit 50 ml Aceton und 10 ml einer 2 n H₂SO₄ versetzt und mit 10%iger wässriger Citronensäure auf pH 7 eingestellt. Man ließ auf Raumtemperatur erwärmen, engte im Vakuum auf ein Restvolumen von 100 ml ein, versetzte mit Wasser und extrahierte mehrmals mit insgesamt 800 ml Dichlormethan. Die vereinigten org. Extrakte wurden mit Wasser gewaschen, über Magnesiumsulfat getrocknet und im Vakuum eingedampft). Man erhielt 13,6 g eines gelben Öles, das unter Druck an Kieselgel mit Ether/Pentan chromatographiert wurde. Man isolierte neben geringen Mengen Ausgangsmaterial 7,43 g (59 %) der Titelverbindung sowie als polarere Komponente 5,12g (41 % ) (1 S, 5 R, 6 S, 7 R) - 6[(E) - (3 R, 4 RS) - 3 - Hydroxy - 4 - methyl - oct - 1 - en - 6 - inyl)] - 7 - benzoyloxy - 2 - oxabicyclo - [3.3.0] octan - 3 - on.
- IR (Film):: 3460, 2970, 2930, 1760, 1720, 1455, 1320, 1270, 1175, 1110, 1070, 740, 715 cm⁻¹.

### Beispiel 2 h

### (1 S, 5 R, 6 S, 7 R) - 6 - [(E) - (4 RS) - 3 - Oxo - 4 - methyl - oct - 1 - en - 6 - inyl] - 7 - benzoyloxy - 2 - oxabicylo [3.3.0] octan - 3 - on

Zu einer Aufschlämmung aus 1,75 g NaH in 190 ml Dimethoxyethan tropfte man unter Feuchtigkeitsausschluß die Lösung von 9,73 g Dimethyl - (2 - oxo - 3 - methyl - hept - 5 - inyl)-phosphonat in 90 ml wasserfreiem Dimethoxyethan. Man ließ noch 30 min bei 23° C reagieren und bei - 45°C die Lösung von 10,0 g Coreylacton in 150 ml Dimethoxyethan innerhalb 50 min zutropfen. Zur Vervollständigung der Reaktion rührte man noch 3 h bei - 20° C nach, versetzte mit 3 ml Ethylacetat, 500 ml Ether und wusch mehrmals mit einer ges. Natriumchloridlösung bis zur Neutralreaktion. Die organische Phase trocknete man über Magnesiumsulfat, filtrierte und engte im Vakuum zur Trockne ein. Man isolierte 13,2 g (98 %) der Titelverbindung als wachsartigen Feststoff.
- IR (CHCl₃):: 2970, 2920, 1775, 1715, 1628, 1450, 1362, 1315, 1270, 1176, 1110, 1070, 980 cm⁻¹.

### Beispiel 3

### (13 E) - (11 R, 15 S, 16 RS) - 6,9 - Di - oxo - 11,15 - dihydroxy - 16 - methyl - 18,18,19,19 - tetradehydro - 13 - prostensäure - methylester

Zu 50 mg (13 E) - (11 R, 15 S, 16 RS) - 6,9 - Di - oxo - 11,15 - dihydroxy-16 - methyl - 18,18,19,19 - tetradehydro - 13 - prostensäure, überschichtet mit 5 ml Ether, gab man unter kräftigem Rühren tropfenweise eine etherische Lösung von Diazomethan, bis sich eine homogene Lösung gebildet hatte. Man dampfte im Vakuum ein und reinigte das erhaltene Rohöl durch präparative Schichtchromatographie an mit Kieselgel beschichteten Glasplatten. Als Laufmittel diente ein Gemisch aus Dichlormethan und Isopropanol. Man isolierte 36 mg (69 %) der Titelverbinung als farbloses Öl.
- IR (Film):: 3380, 2980 - 2820, 1735, 1450, 1440, 1200, 1075, 1025, 975, 908 cm⁻¹.

### Beispiel 4

### (11 R, 15 S, 16 S) - 6,9 - Di - oxo - 11,15 - dihydroxy - 16,20 - dimethyl - 13,14,18,18, 19, 19 - hexadehydro - 13 - prostensäure

410 mg (11 R, 15 S, 16 S) - 6,9 - Di - oxo - 11,15 - bis - (tetrahydropyran - 2 - yloxy) - 16,20 dimethyl - 13,14,18,18,19,19 - hexadehydro - 13 - prostensäure wurden in Analogie zu Beispiel 1 entschützt. Nach chromatographischer Reinigung erhielt man 236 mg (82 %) der Titelverbindung als farbloses Öl.
- IR (Film):: 3400, 3600 bis 2500, 2980, 2230, 1740, 1730, 1710, 1570, 1410, 1280, 1160, 1075, 1020, 970 cm⁻¹.

### Beispiel 5

### (11 R, 15 S, 16 S) - 6,9 - Di - oxo - 11,15 - bis - (tetrahydropyran -2 - yloxyl) - 16,20 - dimethyl - 13,14,18,18,19,19 - hexadehydro - 13 - prostensäure

628 mg (9 S, 11 R, 15 S, 16 S) - 6 - Oxo - 9 - hydroxy - 11,15 - bis - (tetrahydropyran - 2 - yloxy) - 16,20 - dimethyl - 13,14,18,18,19,19 - hexadehydro - 13 - prostensäure oxidierte man analog des in Beispiel 2 aufgeführten Verfahrens mit Jones-Lösung. Nach entsprechend durchgeführter chromatographischer Reinigung wurden 458 mg (73 %) der Titelverbindung als farbloses Öl erhalten.
- IR (Film):: 3600 bis 2500, 2940, 2870, 2230, 1742, 1730, 1700, 1450, 1440, 1380, 1352, 1320 1280, 1260, 1200, 1180, 1150, 1125, 1075, 1034, 1020, 968, 910, 868, 815 cm⁻¹.

Das Ausgansmaterial wurde wie in den Beispielen 5 a bis 5 i beschrieben dargestellt.

### Beispiel 5 a

### (9 S, 11 R, 15 S, 16 S) - 6 - Oxo - 9 - hydroxy - 11,15 - bis - (tetrahydropyran- 2 - yloxy) - 16,20 - dimethyl - 13,14,18,18,19,19 - hexadehydro - 13 - prostensäure

1000 mg (5 RS, 6 RS, 16 S) - 5 - Iod - 16,20 - dimethyl - 13,14,18,18,19,19-hexadehydro - prostaglandin I₁ - 11,15 - bis - ( tetrahydropyranylether) - methylester wurde in völliger Analogie zu Beispiel 2 a umgesetzt und gereinigt. Man isolierte 630 mg (77 %) der Titelverbindung als gelbes Öl.
- IR (Film):: 3500 bis 2500, 2940, 2870, 2230, 1732, 1709, 1450, 1440, 1380, 1354, 1320, 1200, 1117, 1076, 1035, 1020, 970, 908, 870, 815 cm⁻¹.

### Beispiel 5 b

### (5 RS, 6 RS, 16 S) - 5 - Iod - 16,20 - dimethyl - 13,14,18,18,19,19 - hexadehydro - prostaglandin I₁ - 11,15 - bis - (tetrahydropyranylether) - methylester

1,03 g (5 Z) - (9 S, 11 R, 15 S, 16 S) - 9 - Hydroxy - 11,15 - bis - (tetrahydropyran - 2 - yloxy) - 16,20 - dimethyl - 13,14,18,18,19,19 - hexadehydro-prostadiensäure - methylester wurden in Analogie zu Beispiel 2 b umgesetzt. Man isolierte nach der entsprechend durchgeführten Aufarbeitung 1,24 g (98 %) der Titelverbindung als gelbes Öl
- IR (Film):: 2940, 2870, 2230, 1738, 1450, 1438, 1352, 1320, 1200, 1118, 1076, 1035, 1020, 972, 908, 870, 817 cm⁻¹.

### Beispiel 5 c

### (5 Z) - (9 S, 11 R, 15 S, 16 S) - 9 - Hydroxy - 11,15 - bis - (tetrahydropyran - 2 - yloxy) - 16,20 - dimethyl - 13,14,18,18,19,19 - hexadehydroprostadiensäure - methylester

2,39 g (5 Z, 13 E) - (9 S, 11 R, 15 S, 16 S) - 9 - hydroxy - 11,15 - bis - (tetrahydropyran - 2 - yloxy) - 14 - brom - 16,20 - dimethyl - 18,18,19,19 - tetradehydro - prostadiensäure - methylester löste man in einem Gemisch aus 11 ml wasserfreiem THF und 26 ml wasserfreiem Dimethylsulfoxid, versetzte mit 1,23 g Kalium-tertbutanolat und ließ 5 Stunden bei 25° C unter einer Atmosphäre aus trockenem Argon rühren. Man goß in Eiswasser, säuerte mit einer 10%igen Citronensäurelösung an, extrahierte mehrmals mit Diethylether und trocknete die vereinigten organische Extrakte über Magnesiumsulfat. Nach Filtration und Abdampfen des Lösungsmittels im Vakuum isolierte man 2,01 g (99 %) (5 Z) - (9 S, 11 R, 15 S, 16 S) - 9 - Hydroxy - 11,15 - bis - (tetrahydropyran - 2 - yloxy) - 16,20 - dimethyl - 13,14, 18,18,19,19 - hexadehydro - prostadiensäure, die ohne weitere Reinigung mit einer etherischen Lösung von Diazomethan analog zu Beispiel 2 c verestert wurde. Den Rückstand chromatographierte man unter Druck am Kieselgel mit Hexan/Ethylacetat und isolierte 1,22 g (58 %) der Titelverbindung als farbloses Öl.
- IR (Film):: 3500 (breit), 2940, 2870, 2230, 1738, 1452, 1436, 1373, 1354, 1320, 1240, 1200, 1130, 1076, 1020, 970, 908, 870, 816 cm⁻¹.

### Beispiel 5 d

### (5Z, 13 E) - (9 S, 11 R, 15 S, 16 S) - 9 - Hydroxy - 11,15 - bis (tetrahydropyran - 2 - yloxy) - 14 - brom - 16,20 - dimethyl - 18,18,19,19 - tetradehydro - prostadiensäure - methylester

2,53 g (1 S, 3 RS, 5 R, 6 S, 7 R) - 6 - [(E) - (3 S, 4 S) - 2 - Brom - 3 - (tetrahydropyran - 2 - yloxy) - 4 - methyl - non - 1 - en - 6 - inyl] - 7 - (tetrahydropyran - 2 - yloxy) - 2 - oxa - bicyclo [3.3.0] octan - 3-ol wurden in Analogie zu Beispiel 2 c Wittig - Reaktionsbedingungen und anschließenden Veresterungsbedingungen mit Diazomethan unterzogen. Nach chromatographischer Reinigung isolierte man 2,40 g (80 %) der Titelverbindung als farbloses Öl.
- IR (Film):: 3460 (breit), 2940, 2870, 1738, 1650, 1450, 1438, 1374, 1350, 1338, 1320, 1240, 1200, 1128, 1115, 1076, 1052, 1020, 970, 908, 870, 815, 736 cm⁻¹.

### Beispiel 5 e

### (1 S, 3 RS, 5 R, 6 S) - 6 - [(E) - (3 S, 4 S,) - 2 - Brom - 3 - (tetrahydropyran - 2 - yloxy) - 4 - methyl - non - 1 - en - 6 - inyl] - 7 - (tetrahydropyran - 2 - yloxy) - 2 - oxa - bicyclo [3.3.0] octan - 3 - ol

2,77 g (1 S, 3 RS, 5 R, 6 S, 7 R) - 6 - [(E) - 3 S, 4 S) - 2 - Brom - 3 - (tetrahydropyran - 2 - yloxy) - 4 - methyl - non - 1 - en - 6 - inyl] - 7 - (tetrahydropyran - 2 - yloxy) - 2 - oxa - bicyclo [3.3.0] octan - 3 - on wurden in Analogie zu Beispiel 2 d reduziert. Nach Aufarbeitung und chromatographischer Reinigung isolierte man 2,54 g (91 %) der Titelverbindung als farbloses Öl.
- IR (Film):: 3400 (breit), 2940, 2870, 1736, 1648, 1452, 1440, 1375, 1352, 1340, 1322, 1260, 1200, 1184, 1120, 1070, 1020, 970, 908, 868, 815 cm⁻¹.

### Beispiel 5 f

### (1 S, 5 R, 6, S, 7 R) - 6 - [(E) - (3 S, 4 S) - 2 - Brom - 3 - (tetrahydropyran - 2 - yloxy) - 4 - methyl - non - 1 - en - 6 - inyl] - 7 - (tetrahydropyran - 2 - yloxy) - 2 - oxa - bicyclo [3.3.0] octan - 3 - on

2,00 g (1 S, 5 R, 6 S, 7 R) - 6 - [(E) - (3 S, 4 S) - 2 - Brom - 3 - hydroxy - 4 - methyl - non - 1 - en - 6 - inyl] - 7 - hydroxy - 2 - oxa - bicyclo [3.3.0] octan - 3 - on wurde in Analogie zu Beispiel 2 e umgesetzt. Nach chromatographischer Reinigung isolierte man 2,78 g (96 %) der Titelverbindung als farbloses Öl.
- IR (Film):: 2970, 2930, 1770, 1640, 1450, 1430, 1360, 1335, 1235, 1120, 1070, 1025, 910, 868, 812 cm⁻¹.

### Beispiel 5 g

### (1 S, 5 R, 6 S, 7 R) - 6 - [(E) - (3 S, 4 S) - 2 - Brom - 3 - hydroxy - 4 - methyl - non - 1 - en - 6 - inyl] - 7 - hydroxy - 2 - oxa - bicyclo [3.3.0] octan - 3 - on

3,12 g (1 S, 5 R, 6 S, 7 R) - 6 - [(E) - (3 S, 4 S) - 2 - Brom - 3 - hydroxy - 4 - methyl - non - 1 - en - 6 - inyl] - 7 - benzoyloxy - 2 - oxa - bicylo [3.3.0] octan - 3 - on löste man in 18 ml p.A. Methanol, versetzte mit 290 mg fein gepulvertem Kaliumcarbonat und ließ 3 h bei 25° C rühren. Durch Zugabe einer 50%igen Salzsäure stellte man auf pH-7 ein und engte bei 30°C im Wasserstrahlvakuum ein. Der Rückstand wurde in Methylenchlorid aufgenommen, über Magnesiumsulfat und Celite filtriert, erneut im Wasserstrahlvakuum eingeengt und unter Druck an ca. 200 ml feinem Kieselgel unter Verwendung eines Gradienten aus Hexan/Essigester chromatographiert. Man isolierte 2,00 g (82 %) der Titelverbindung als farbloses Öl.
- IR (Film):: 3400, 2950, 2910, 1755, 1640, 1440, 1415, 1340, 1300, 1180, 1075, 1030, 968, 905 cm⁻¹.

### Beispiel 5 h

### (1 S, 5 R, 6 S, 7 R) - 6 - [(E) - (3 S, 4 S) - 2 - Brom - 3 - hydroxy - 4 - methyl - non - 1 - en - 6 - inyl] - 7 - benzoyloxy - 2 - oxa - bicyclo [3.3.0] octan - 3 - on

16,7 g (1 S, 5 R, 6 S, 7 R) - 6 - [(E) - (4 S) - 2 - Brom - 3 - oxo - 4 - methyl - non - 1 - en - 6 - inyl] - 7 - benzoyloxy - 2 - oxa - bicyclo [3.3.0] octan - 3 - on, wurden in Analogie zu Beispiel 2 g reduziert. Nach chromatographischer Reinigung isolierte man 4,1 g (24 %) der Titelverbindung sowie 6,6 g (1 S, 5 R, 6 S, 7 R) - 6 - [(E) - (3 S, 4 S) - 2 - Brom - 3 - oxo - 4 - methyl - non - 1 - en - 6 - inyl] - 7 - benzoyloxy - 2 - oxa - bicyclo [3.3.0] octan - 3 - on (39 %).
- IR (Film):: 3460 (breit), 3060, 2970, 2930, 1770, 1714, 1602, 1450, 1317, 1272, 1178, 1115, 1070, 1026, 737, 715 cm⁻¹

### Beispiel 5 i

### (1 S, 5 R, 6 S, 7 R) - 6 - [(E) - (4 S) - 2 - Brom - 3 - oxo - 4 - methyl - non - 1 - en - 6 - inyl] - 7 - benzoyloxy - 2 - oxa - bicyclo [3.3.0]octan - 3 - on

Zu einer Aufschlämmung aus 2,58 g NaH in 225 ml Dimethoxyethan tropfte man unter Feuchtigkeitsausschluß die Lösung von 13,7 g Dimethyl - [(3 S) - 2 - oxo - 3 - methyl - oct - 5 - inyl] phosphonat in 135 ml Dimethoxyethan bei 0° C zu. Nach 20 minutigem Rühren versetzte man die nun klare Lösung mit 9,89 g fein pulverisiertem N-Bromsuccinimid, rührte 1 h bei 0°C nach, tropfte die Lösung aus 12,3 g Coreylacton zu und ließ weitere 2 h bei 0° C reagieren. Man ließ unter kräftigem Rühren in 800 ml eine 10%ige wässrige Ammonchloridlösung einlaufen, extrahierte mehrmals mit insgesamt 1,5 l Diethylether, wusch die org. Phase mit Wasser nach, trocknete über Magnesiumsulfat und isolierte nach Filtration und Einengen im Vakuum 27,4 g eines gelben Rohöls, das unter Druck mittels eines Gradienten aus Hexan und Essigester chromatographisch gereinigt wurde. Man isolierte 16,9 g (72 %) der Titelverbindung als farbloses Öl
- IR (Film):: 2970, 2920, 1765, 1720, 1600, 1450, 1360, 1315, 1270, 1170, 1105, 1070, 965 cm⁻¹.

### Beispiel 6

### (11 R, 15 S, 16 S) - 6,9 - Di - oxo - 11,15 - dihydroxy - 16,20 - dimethyl - 13,14,18,18,19,19 - hexadehydro - 13 - prostensäure - methylester

Zu einer kräftig gerührten Emulsion aus 63 mg (11 R, 15 S, 16 S) - 6,9 - Di - oxo - 11,15 - dihydroxy - 16,20 - dimethyl - 13,14,18,18,19,19 - hexadehydro - 13 - prostensäure in 10 ml Ether tropfte man bei 0 bis 5° C eine etherische Lösung von Diazomethan. Die Aufarbeitung und Reinigung erfolgte in Analogie zu Beispiel 3. Isoliert wurden 46 mg (70 %) der Titelverbindung als farbloses Öl.
- IR (Film):: 3400, 2980 - 2820, 2230, 1737, 1450, 1440, 1200, 1078, 1020, 970, 910 cm⁻¹.

## Patentansprüche

1. 6-Oxo-prostaglandin-E₁-derivate der Formel I, 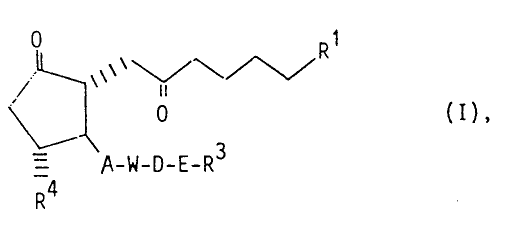 worin
R¹ den Rest COOR² mit R² in der Bedeutung eines Wasserstoffatomes, einer C₁₋₁₀-Alkyl-, einer gegebenenfalls durch Alkylgruppen mit 1-4 C-Atomen substituierten C₅-C₆-Cycloalkyl- oder einer C₆-C₁₀-Aryl-Gruppe oder eines heterocyclischen Restes, oder den Rest CONHSO₂R⁵ mit R⁵ als C₁₋₁₀-Alkyl-, gegebenenfalls durch Alkylgruppen mit 1-4 C-Atomen substituierte C₅₋₆-Cycloalkyl-oder C₆₋₁₀-Aryl-.Gruppe,
A eine E-konfigurierte CH=CH-oder eine -C≡C-Gruppe,
W eine freie oder funktionell abgewandelte 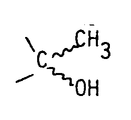 Hydroxymethylengruppe oder eine freie oder funktionell abgewandelte -Gruppe, wobei die OH-Gruppe jeweils α- oder β-ständig sein kann,
D eine geradkettige oder verzweigtkettige Alkylengruppe mit 1-5 C-Atomen,
E eine -C≡C-Gruppe in 18,19- oder 19,20-Position,
R³ eine gesättigte oder ungesättigte C₁-C₁₀-Alkyl-,gegebenenfalls durch Alkylgruppen mit 1-4 C-Atomen substituierte C₃-C₁₀-Cycloalkyl-oder eine gegebenenfalls substituierte C₆-6₁₀-Arylgruppe oder eine heterocyclische Gruppe,
R⁴ eine freie oder funktionell abgewandelte Hydroxygruppe, wobei in 16- und/oder 20-Position Methyl steht und falls R² die Bedeutung eines Wasserstoffatoms hat, deren Salze mit physiologisch verträglichen Basen bedeuten sowie die α-, β- oder γ-Cyclodextrinclathrate der Verbindungen der Formel I.

2. Verfahren zur Herstellung der Verbindungen der Formel I, dadurch gekennzeichnet, daß man eine verbindung der Formel II, 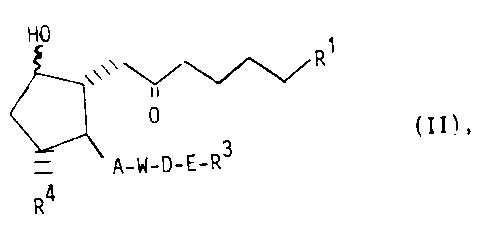 worin R¹, R³, R⁴, A, W, D und E die oben angegebenen Bedeutungen aufweisen und freie OH-Gruppen in R⁴ und W geschützt sind, mit Chromschwefelsäure, Pyridiniumdichromat, Pyridiniumchlorochromat, Collins-Reagenz oder mit den Komplexen aus CrO₃ und anderen Aminbasen oxidiert und geschützte Hydroxygruppen freisetzt und/oder freie Hydroxygruppen verestert, verethert und/oder eine veresterte Carboxylgruppe verseift oder eine Carboxylgruppe mit einer physiologisch verträglichen Base in ein Salz überführt oder mit α-, β- oder γ-Cyclodextrin zu einem Clathrat umsetzt.

3. Arzneimittel, bestehend aus einer oder mehreren Verbindungen der Formel I und üblichen Hilfs- und Trägerstoffen.

## Claims

1. 6-oxo-prostaglandin E₁ derivatives of formula I 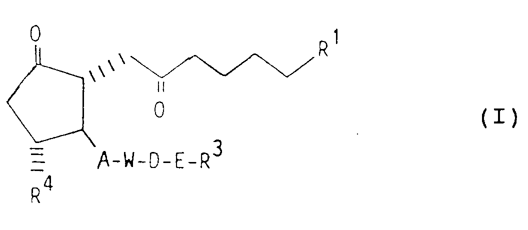 wherein
R¹ is the radical COOR² wherein R² is a hydrogen atom, a C₁-C₁₀alkyl group, a C₅-C₆cycloalkyl group optionally substituted by alkyl groups having from 1 to 4 carbon atoms, or a C₆-C₁₀aryl group or a heterocyclic radical, or R¹ is the radical CONHSO₂R⁵ wherein R⁵ is a C₁-C₁₀alkyl group, a C₅-C₆cycloalkyl group optionally substituted by alkyl groups having from 1 to 4 carbon atoms, or a C₆-C₁₀aryl group,
A is an E-configuration CH=CH- or a -C≡C- group,
W is a free or functionally-modified hydroxymethylene group or a free or functionally-modified 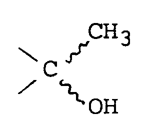 group wherein the OH group in each case may be in the α- or β-configuration,
D is a straight-chain or branched-chain alkylene group having from 1 to 5 carbon atoms,
E is a -C≡C- group in the 18,19- or 19,20-position,
R³ is a saturated or unsaturated C₁-C₁₀alkyl group, a C₃-C₁₀cycloalkyl group optionally substituted by alkyl groups having from 1 to 4 carbon atoms, or an optionally substituted C₆-C₁₀aryl group, or a heterocyclic group,
R⁴ is a free or functionally-modified hydroxy group, there being a methyl group in the 16- and/or 20-position(s), and, when R² is a hydrogen atom, the salts thereof with physiologically tolerable bases, and the α-, β- or γ-cyclodextrin clathrates of compounds of formula I.

2. A process for the preparation of the compounds of formula I, characterised in that a compound of formula II 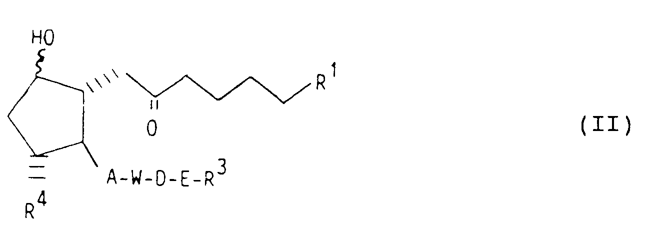 wherein R¹, R³, R⁴, A, W, D and E have the meanings given above and free OH groups in R⁴ and W are protected, is oxidised with chromosulphuric acid, pyridinium dichromate, pyridinium chlorochromate, Collins reagent or with the complexes of CrO₃ with other amine bases, and protected hydroxy groups are freed and/or free hydroxy groups are esterified or etherified, and/or an esterified carboxy group is hydrolysed or a carboxy group is converted with a physiologically tolerable base into a salt or reacted with α-, β- or γ-cyclodextrin to form a clathrate.

3. Medicaments consisting of one or more compounds of formula I and customary excipients and carriers.

## Revendications

1. Dérivés de la 6-oxo-prostaglandine-E₁ de formule I : 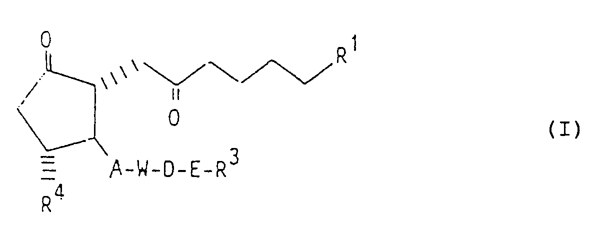 dans laquelle
R¹ représente un radical COOR², R² étant un atome d'hydrogène, un alkyle en C₁-C₁₀, un cycloalkyle en C₅-C₆ éventuellement substitué par des alkyles en c₁-C₄ ou un aryle en C₆-C₁₀, ou bien un radical hétérocyclique ou un radical CONHSO₂R⁵, R⁵ étant un alkyle en C₁-C₁₀, un cycloalkyle en C₅-C₆ éventuellement substitué par des alkyles en C₁-C₄ ou un aryle en C₆-C₁₀,
A représente un groupe -CH=CH- ou -C≡C- de configuration E,
W un groupe hydroxyméthylène libre ou à fonction modifiée ou bien un groupe 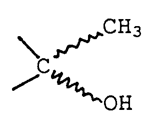 libre ou a fonction modifiée, dont le groupe OH peut être à la position α ou β,
D un alkylène à chaîne linéaire ou ramifiée ayant de 1 à 5 atomes de carbone,
E un groupe -C≡C- à la position 18,19 ou 19,20,
R³ un alkyle en C₁-C₁₀ saturé ou insaturé, un cycloalkyle en C₃-C₁₀ éventuellement substitué par des alkyles en C₁-C₄ ou encore un aryle en C₆-C₁₀ éventuellement substitué ou un radical hétérocyclique, et
R⁴ un groupe hydroxylique libre ou à fonction modifiée, un groupe méthyle occupant la position 16 et/ou 20, et si R² est un atome d'hydrogène, les sels de ces composés de formule I formés avec des bases physiologiquement compatibles et tolérées ainsi que leurs clathrates avec la cyclodextrine α, β ou γ.

2. Procédé de préparation des composés de formule I selon la revendication 1, procédé caractérisé en ce que l'on oxyde un composé de formule II : 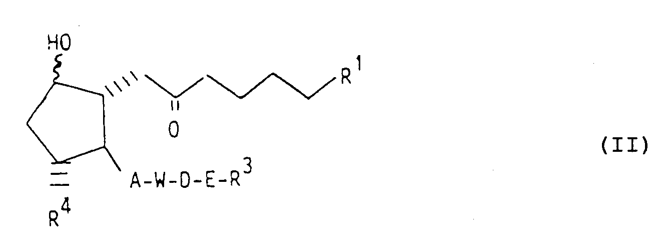 les divers symboles ayant les significations indiquées à la revendication 1, et des hydroxyles libres de R⁴ et W étant protégés, avec de l'acide sulfochromique, du dichromate de pyridinium, du chlorochromate de pyridinium, le réactif de Collins ou encore avec les complexes de CrO₃ et d'autres bases amines, puis on libère les hydroxyles protégés et/ou estérifie ou éthérifie les hydroxyles libres et/ou on saponifie un groupe carboxylique estérifié ou on salifie un groupe carboxylique avec une base physiologiquement compatible ou encore on fait réagir le composé formé avec la cyclodextrine α, β ou γ pour obtenir le clathrate correspondant.

3. Médicaments constitués d'un ou de plusieurs composés de formule I de la revendication 1 avec des adjuvants et véhicules courants.
